# EUROPEAN PATENT APPLICATION

(11) **EP 4 623 897 A1**
(43) Date of publication of application: **01.10.2025**
(21) Application number: 23894954.9
(22) Date of filing: 20.11.2023
(51) Int. Cl.: A61K 9/00, A61K 47/12, A61K 31/351, A61P 29/00

(54) **PHARMACEUTICAL COMPOSITION CONTAINING HIGH CONCENTRATION OF OPIRANSERIN**

(30) Priority: 21.11.2022 KR 20220156393
(71) Applicant: Vivozon, Inc., Yongin-si Gyeonggi-do 16914 (KR)
(72) Inventor: LEE, Sung Il, Yongin-si Gyeonggi-do 16914 (KR); CHOI, Jung Sub, Yongin-si Gyeonggi-do 16914 (KR)
(74) Representative: Vossius & Partner Patentanwälte Rechtsanwälte mbB
(86) International application number: PCT/KR2023/018645
(87) International publication number: WO 2024/112026

(57) **Abstract**

A pharmaceutical composition containing a high concentration of opiranserin is disclosed. More specifically, disclosed is a pharmaceutical composition comprising: opiranserin or a pharmaceutically acceptable salt thereof; a carrier selected from sodium benzoate, trisodium citrate, sodium salicylate and mixtures thereof; and a solvent for injection.

## Description

### TECHNICAL FIELD

Disclosed is a pharmaceutical composition comprising a high concentration of opiranserin. More specifically, disclosed is a pharmaceutical composition comprising opiranserin or a pharmaceutically acceptable salt thereof; a carrier selected from sodium benzoate, trisodium citrate, sodium salicylate and a mixture thereof; and a solvent for injection.

### BACKGROUND ART

Many of the drugs currently used as medicines are poorly soluble and show low bioavailability when administered to a subject due to low solubility. Various formulation methods are being studied to solubilize poorly soluble drugs. However, cases of actual application in drug development are rare due to insignificant effect or limitations in formulation and usage.

Formulation technologies to improve drug solubility can be largely divided into physical modifications and chemical modifications. Examples of physical modification include a method of reducing the size of drug particles, a method of modifying the crystallization of polymorphs, a method of dispersing drugs in carriers such as eutectic mixtures or solid dispersions, a method of complexation by the use of complexing agents, and solubilization methods by surfactants using microemulsions and self-microemulsifying drug delivery systems (SMEDDS). Examples of chemical modification include a method of improving drug solubility by adjusting pH or using salts.

### [Patent documents]

US Patent No. 9,359,346

International Publication No. WO 2020/256456

### DISCLOSURE OF INVENTION

### TECHNICAL PROBLEM

One object of the present invention is the provision of a pharmaceutical composition comprising a high concentration of opiranserin.

Another object of the present invention is the provision of a method for quickly and safely preparing a bolus intravenous infusion composition by using the pharmaceutical composition.

### SOLUTION TO PROBLEM

To solve the above technical problem, there is provided a pharmaceutical composition comprising opiranserin or a pharmaceutically acceptable salt thereof; a carrier selected from sodium benzoate, trisodium citrate, sodium salicylate and a mixture thereof; and a solvent for injection.

In addition, there is provided a pre-filled syringe containing the pharmaceutical composition.

Furthermore, there is provided a method for preparing a bolus intravenous infusion composition comprising: preparing an intravenous administration container (IV container) containing saline solution; and injecting the pharmaceutical composition into the intravenous administration container (IV container) one time.

### EFFECTS OF THE INVENTION

According to the present invention, a pharmaceutical composition comprising a high concentration of opiranserin can be prepared. The pharmaceutical composition comprising a high concentration of opiranserin according to the present invention can be stored and transported in a small volume, and can be used to quickly and safely prepare a bolus intravenous infusion composition, thereby providing convenience to medical staffs and patients.

### BRIEF DESCRIPTION OF DRAWINGS

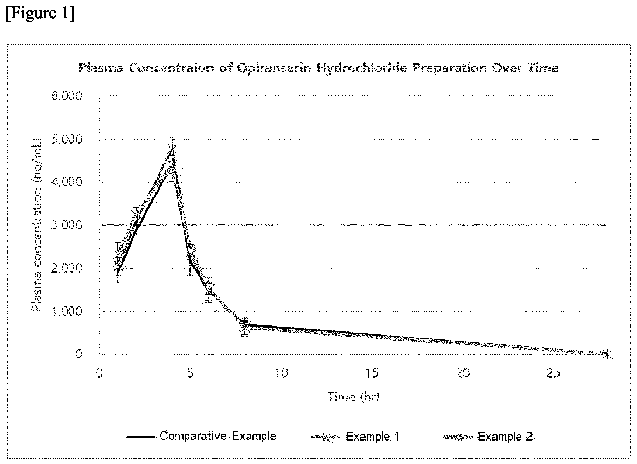

Figure 1 represents a graph showing the results of analyzing plasma concentration measured in Experimental Example 6.

### MODE FOR THE INVENTION

The present invention is described in detail hereinafter.

According to one aspect of the present invention, there is provided a pharmaceutical composition comprising opiranserin or a pharmaceutically acceptable salt thereof; a carrier selected from sodium benzoate, trisodium citrate, sodium salicylate and a mixture thereof; and a solvent for injection.

Opiranserin used herein is 4-butoxy-N-((4-(dimethylamino)tetrahydro-2H-pyran-4-yl)methyl)-3,5-dimethoxy benzamide of the following Formula 1:

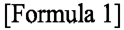

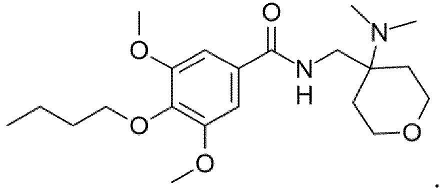

Opiranserin is also known as VVZ-149. The method of synthesizing opiranserin, its use for treating postoperative pain, and reducing an analgesic need in treating postoperative pain are disclosed in US Patent No. 9,359,346 and International Publication No. WO 2020/256456, and the contents of which are incorporated herein by reference.

In one embodiment according to the present invention, the pharmaceutically acceptable salt of opiranserin may be hydrochloride, phosphate or sulfate. In another embodiment according to the present invention, the pharmaceutically acceptable salt of opiranserin may be hydrochloride. Opiranserin hydrochloride has a solubility of 25 mg/mL, which can be regarded as "sparingly soluble." Opiranserin hydrochloride is currently used clinically as an injection at a concentration of 10 mg/mL. For adult patients (approximately 60 kg), 100 mL of an injection with a concentration of 10 mg/mL of opiranserin is diluted in normal saline, and it is injected in the form of an infusion.

In another embodiment according to the present invention, the content of opiranserin or a pharmaceutically acceptable salt thereof may be 0.1 mol/L to 0.7 mol/L based on the total volume of the composition. In another embodiment according to the present invention, the content of opiranserin or a pharmaceutically acceptable salt thereof may be 0.2 mol/L to 0.7 mol/L based on the total volume of the composition.

In the pharmaceutical composition according to the present invention, in order to prepare a composition comprising a high concentration of poorly soluble opiranserin, a carrier selected from sodium benzoate, trisodium citrate, sodium salicylate and a mixture thereof is used. In another embodiment according to the present invention, the carrier may be hydrotrope. Hydrotropes are amphiphilic low-molecular-weight compounds that are easily soluble in aqueous solutions. It has been suggested that some hydrotropes improve the solubility of drugs through non-covalent self-aggregation in non-polar microregions above the minimal hydrotrope concentration (MHC). Hydrotropes consist of a lipophilic part and a hydrophilic part similar to surfactants, but the lipophilic part is relatively shorter than that of surfactants and has a cyclic or branched shape. In addition, hydrotropes are not classified as a surfactant because they do not form micelles. Hydrotropes are mainly used commercially in functional cosmetics and car cleaners. Examples of hydrotropes include nicotinamide, N,N-diethyl nicotinamide, sodium benzoate, benzyl benzoate, sodium 4-aminobenzoate, sodium salicylate, resorcinol, piperazine, 2-butoxyethyl sulfonic acid sodium salt, lysine, urea, sodium citrate, trisodium citrate, and the like.

In the pharmaceutical composition according to the present invention, the amount of carrier (hydrotrope) can be adjusted depending on the components of the hydrotrope used, the allowed amount of hydrotrope administered to a subject and the content of opiranserin.

In another embodiment according to the present invention, the content of sodium benzoate may be 0.2 mol/L to 2.0 mol/L, and more specifically 0.26 mol/L to 0.90 mol/L, based on the total volume of the composition. In another embodiment according to the present invention, the content of the added sodium benzoate may be 30 mg/mL to 300 mg/mL, and more specifically 37 mg/mL to 130 mg/mL, based on the total volume of the prepared composition to be prepared.

In another embodiment according to the present invention, the content of trisodium citrate may be 0.08 mol/L to 0.35 mol/L based on the total volume of the composition. The trisodium citrate may be added, for example, in the form of dihydrate, and the content of the added trisodium citrate dihydrate may be 25.0 mg/mL to 100 mg/mL based on the total volume of the composition to be prepared.

In another embodiment according to the present invention, the content of sodium salicylate may be 0.015 mol/L to 0.10 mol/L based on the total volume of the composition. In another embodiment according to the present invention, the content of the added sodium salicylate may be 2.5 mg/mL to 16 mg/mL based on the total volume of the composition to be prepared.

In another embodiment according to the present invention, the content of the carrier (hydrotrope) may be determined as the molar ratio with opiranserin or a pharmaceutically acceptable salt thereof. In another embodiment according to the present invention, the molar ratio of opiranserin or a pharmaceutically acceptable salt thereof to sodium benzoate may be 1 : 0.5 to 3.0, specifically 1 : 1.0 to 2.4, more specifically 1 : 1.2 to 1.6, and even more specifically 1 : 1.3 to 1.5. In another embodiment according to the present invention, the molar ratio of opiranserin or a pharmaceutically acceptable salt thereof to sodium benzoate may be 1 : 1.4 to 1.6, and specifically 1 : 1.48 to 1.49.

By using a carrier (hydrotrope) selected from sodium benzoate, trisodium citrate, sodium salicylate and a mixture thereof, in the pharmaceutical composition according to the present invention the content of opiranserin or a pharmaceutically acceptable salt thereof can be secured to 0.1 mol/L or more, specifically 0.2 mol/L or more, more specifically 0.4 mol/L or more, and even more specifically 0.7 mol/L based on the total volume of the composition. In another embodiment according to the present invention, opiranserin hydrochloride can be dissolved in the pharmaceutical composition in an amount of 50 mg/mL or more, 100 mg/mL or more, 200 mg/mL or more, or up to 300 mg/mL based on the total volume of the composition, and stability can be ensured when stored at room temperature and/or in refrigerated condition.

In another embodiment according to the present invention, the solvent for injection may be water for injection.

In another embodiment according to the present invention, the pharmaceutical composition may further comprise one or more pharmaceutically acceptable excipients. Examples of excipients include, but are not limited to, surfactants, antioxidants, pH adjusting agents, osmotic agents, acidifying agents, alkalinizing agents, preservatives, buffers, chelating agents, stabilizers, emulsifiers and/or solubilizers.

Each of the above excipients constitutes an individual embodiment and may be added to any claim in any suitable combination. For example, in another embodiment according to the present invention, the pharmaceutical composition may further comprise a surfactant to enhance refrigeration stability. Specific examples of surfactants include polysorbate 20, polysorbate 80, polyoxyl 35 castor oil, polyoxyl 15 hydroxystearate, or a mixture thereof, but are not limited thereto. In another embodiment according to the present invention, the content of the surfactant may be 1 mg/mL to 50 mg/mL based on the total volume of the composition.

In another embodiment according to the present invention, the pharmaceutical composition may be prepared by the steps of: a) dissolving a carrier (hydrotrope) selected from sodium benzoate, trisodium citrate, sodium salicylate and a mixture thereof in water for injection to obtain a solvent system; b) preparing a solution by adding opiranserin or a pharmaceutically acceptable salt thereof to the solvent system; and c) performing aseptic filtration.

According to another aspect of the present invention, there is provided a pharmaceutical composition as an analgesic injection, which comprises 0.1 mol/L to 0.7 mol/L of opiranserin or a pharmaceutically acceptable salt thereof based on the total volume of the injection.

According to another aspect of the present invention, there is provided a pre-filled syringe containing the pharmaceutical composition according to the present invention. In another embodiment according to the present invention, the pharmaceutical composition can be used as an analgesic injection, and can be used in the form of a pre-filled syringe in which a single dose is pre-filled into a syringe.

According to another aspect of the present invention, there is provided a method for preparing a bolus intravenous infusion composition comprising: preparing an intravenous administration container (IV container) containing saline solution; and injecting the pharmaceutical composition according to the present invention into the intravenous administration container (IV container) one time.

According to another aspect of the present invention, there is provided a method for preparing a bolus intravenous infusion composition comprising: preparing an intravenous administration container (IV container) containing saline solution; and injecting the pharmaceutical composition filled in the pre-filled syringe into the intravenous administration container (IV container) one time.

### Examples

Hereinafter, the present invention is explained in more detail with the following examples. However, it must be understood that the protection scope of the present invention is not limited to the examples.

### Experimental Example 1: Solubility measurement of opiranserin hydrochloride according to solvents

An excess amount of opiranserin hydrochloride was added to the solvents shown in Table 1, stirred under the following conditions, centrifuged and filtered, and the filtrate was diluted. The solubility of the drug was measured using high-performance liquid chromatography (HPLC).

**[Table 1]**

| No. | Stirring temperature (°C) / Stirring time | Solvent type and content | Opiranserin hydrochloride Solubility (mg/mL) |
|---|---|---|---|
| Solvent 1 | 25°C / 7 days | Purified water | 45.19 |
| Solvent 2 | 25°C / 7 days | 100% Ethanol | 25.11 |
| Solvent 3 | 25°C / 7 days | 100% Methanol | 36.00 |
| Solvent 4 | 25°C / 7 days | 100% Propylene glycol | 13.03 |
| Solvent 5 | 25°C / 7 days | 100% Polyethylene | 2.59 |
| Solvent 6 | 25°C / 7 days | 1 w/v% Polyoxyl 35 castor oil | 42.06 |
| Solvent 7 | 25°C / 7 days | 1 w/v% Caprylocaproyl polyoxyl 8 glyceride | 46.16 |
| Solvent 8 | 25°C / 7 days | 1 w/v% Macrogol 15 hydroxystearate | 43.98 |
| Solvent 9 | 25°C / 7 days | 1 w/v% Polysorbate 80 | 44.73 |
| Solvent 10 | 25°C / 7 days | 1 w/v% Hydroxpropyl-betacyclodextrin | 49.17 |
| Solvent 11 | 25°C / 7 days | 3 w/v% Hydroxpropyl-betacyclodextrin | 55.63 |
| Solvent 12 | 25°C / 7 days | 6 w/v% Hydroxpropyl-betacyclodextrin | 63.30 |
| Solvent 13 | 25°C / 7 days | 1 w/v% Triacetin | 49.66 |
| Solvent 14 | 25°C / 7 days | 3 w/v% Triacetin | 57.05 |
| Solvent 15 | 25°C / 7 days | 6 w/v% Triacetin | 72.69 |
| Solvent 16 | 25°C / 7 days | 1 w/v% PEG-6 caprylic/capric glyceride | 49.00 |
| Solvent 17 | 25°C / 7 days | 3 w/v% PEG-6 caprylic/capric glyceride | 56.98 |
| Solvent 18 | 25°C / 7 days | 6 w/v% PEG-6 caprylic/capric glyceride | 67.59 |
| Solvent 19 | 40°C / 3 hours | 1 w/v% Triacetin | 78.6 |
| Solvent 20 | 40°C / 3 hours | 6 w/v% Triacetin | 86.1 |
| Solvent 21 | 40°C / 3 hours | 1 w/v% Poloxamer 124 | 72.1 |
| Solvent 22 | 40°C / 3 hours | 5 w/v% Poloxamer 124 | 83.8 |
| Solvent 23 | 40°C / 3 hours | 1 w/v% Propylene glycol | 61.8 |
| Solvent 24 | 40°C / 3 hours | 5 w/v% Propylene glycol | 74.0 |

### Experimental Example 2: Room temperature stability of dissolved opiranserin hydrochloride

Among solvents showing high solubility in Experimental Example 1, the solvents that have no irritation or can minimize irritation when preparing medicines were selected as shown in Table 2. Then, the solubility and short-term room temperature stability of opiranserin hydrochloride according to the concentration content were estimated. Each preparation was prepared by adjusting the solvent according to the concentration, adding an excess amount of opiranserin hydrochloride, and stirring at 40°C for 3 hours. The preparation was centrifuged, the supernatant was transferred to a glass vial, and stored at room temperature to observe properties and analyze content. Liquid chromatography (HPLC) was used to analyze the content of opiranserin hydrochloride, and sampling was carried out upon completion of preparation and after storage at room temperature for 65 hours.

**[Table 2]**

| No. | Solvent type and content | Opiranserin hydrochloride concentration (mg/mL) | | Change in properties after storage at room temperature for 65 hours |
|---|---|---|---|---|
| | | Initial | After storage at room temperature for 65 hours | |
| Solvent 25 | 1 w/v% Triacetin | 78.6 | 44.33 | Recrystallization generation |
| Solvent 26 | 6 w/v% Triacetin | 86.1 | 38.1 | Recrystallization generation |
| Solvent 27 | 1 w/v% Poloxamer 124 | 72.1 | 41.1 | Recrystallization generation |
| Solvent 28 | 5 w/v% Poloxamer 124 | 83.8 | 42.5 | Recrystallization generation |
| Solvent 29 | 1 w/v% Propylene glycol | 61.8 | 34.8 | Recrystallization generation |
| Solvent 30 | 5 w/v% Propylene glycol | 74.0 | 34.8 | Recrystallization generation |

In Experimental Example 1, the opiranserin hydrochloride preparation dissolved in 6 w/v% triacetin solvent (Solvent 20) was dissolved up to about 86 mg/mL. However, during the short-term room temperature stability test, recrystallization of needle-shaped structure was generated, and at this time, it was confirmed that the solubility of opiranserin hydrochloride in the preparation was reduced to 38.1 mg/mL.

From the above results, it was confirmed that it is difficult to prepare opiranserin hydrochloride solution at a high concentration and maintain stability at the desired level using only conventional surfactants or excipients that help solubilization.

### Experimental Example 3: Screening of hydrotropes for improving solubility of opiranserin hydrochloride

With hydrotropes that can be used in medicines, screening was conducted as shown in Table 3 below. After adding 50 mL of water for injection into a 200 mL beaker, a weight of hydrotrope corresponding to the target content was added thereto and stirred at room temperature to completely dissolve it. Then, a weight of opiranserin hydrochloride corresponding to the target concentration was added and stirred at room temperature to completely dissolve it. If it was not completely dissolved in some solvents, the temperature was raised to 40°C and additional stirring was carried out. When the added materials were completely dissolved, water for injection was added to the beaker to reach the 100 mL mark and further stirred to ensure that the composition was completely mixed. The prepared compositions were filtered using a 0.2 µm syringe filter and then dispensed into vials for injection.

**[Table 3]**

| Composition | Hydrotrope | | | Opiranserin hydrochloride | | Stirring temperature (°C) / Stirring time | Opiranserin hydrochloride dissolved or not (Naked eye appearance) |
|---|---|---|---|---|---|---|---|
| | Component | Conc. (mg/mL) | Conc. (g/L) | Conc. (mg/mL) | Conc. (mol/L) | | |
| Composition 1 | Sodium benzoate | 288 | 1.998 | 100 | 1.998 | 25°C/ 1 hour | Dissolved |
| Composition 2 | | 30 | 0.208 | 100 | 0.208 | 25°C/ 1 hour | Dissolved |
| Composition 3 | | 100 | 0.694 | 100 | 0.694 | 25°C/ 1 hour | Dissolved |
| Composition 4 | | 288 | 1.998 | 200 | 1.998 | 25°C/ 1 hour | Dissolved |
| Composition 5 | | 100 | 0.694 | 150 | 0.694 | 25°C/ 1 hour | Dissolved |
| Composition 6 | | 100 | 0.694 | 200 | 0.694 | 25°C/ 1 hour | Dissolved |
| Composition 7 | | 100 | 0.694 | 250 | 0.694 | 25°C/ 1 hour + 40°C/1 hour | Dissolved |
| Composition 8 | | 100 | 0.694 | 300 | 0.694 | 25°C/ 1 hour + 40°C/1 hour | Dissolved |
| Composition 9 | Trisodium citrate dihydrate | 25 | 0.085 | 100 | 0.085 | 25°C/ 1 hour | Dissolved |
| Composition 10 | | 50 | 0.170 | 100 | 0.170 | 25°C/ 1 hour | Dissolved |
| Composition 11 | | 100 | 0.34 | 100 | 0.34 | 25°C/ 1 hour | Dissolved |
| Composition 12 | | 250 | 0.850 | 100 | 0.850 | 25°C/ 1 hour + 40°C/1 hour | Recrystallization after dissolution |
| Composition 13 | | 25 | 0.085 | 150 | 0.085 | 25°C/ 1 hour + 40°C/1 hour | Recrystallization after dissolution |
| Composition 14 | | 25 | 0.085 | 200 | 0.085 | 25°C/ 1 hour + 40°C/1 hour | Recrystallization after dissolution |
| Composition 15 | | 25 | 0.085 | 250 | 0.085 | 25°C/ 1 hour + 40°C/1 hour | Recrystallization after dissolution |
| Composition 16 | | 25 | 0.085 | 300 | 0.085 | 25°C/ 1 hour + 40°C/1 hour | Recrystallization after dissolution |
| Composition 17 | Sodium salicylate | 16 | 0.0999 | 100 | 0.0999 | 25°C/ 1 hour | Dissolved |
| Composition 18 | | 2.5 | 0.0156 | 100 | 0.015 | 25°C/ 1 hour | Dissolved |
| Composition 19 | | 16 | 0.100 | 150 | 0.100 | 25°C/ 1 hour + 40°C/1 hour | Does not dissolve |
| Composition 20 | | 16 | 0.100 | 200 | 0.100 | 25°C/ 1 hour + 40°C/1 hour | Does not dissolve |
| Composition 21 | | 16 | 0.100 | 250 | 0.100 | 25°C/ 1 hour + 40°C/1 hour | Does not dissolve |
| Composition 22 | | 16 | 0.100 | 300 | 0.100 | 25°C/ 1 hour + 40°C/1 hour | Does not dissolve |

As can be seen from Table 3, when sodium benzoate was used as a hydrotrope, up to 300 mg/mL of opiranserin hydrochloride could be dissolved. When trisodium citrate dihydrate was used as a hydrotrope, opiranserin hydrochloride could be stably dissolved up to 100 mg/mL. When sodium salicylate was used as a hydrotrope, opiranserin hydrochloride could be stably dissolved up to 100 mg/mL.

### Experimental Example 4: Dissolution stability according to molar ratio of opiranserin hydrochloride and sodium benzoate

In order to evaluate the dissolution stability of opiranserin hydrochloride according to the content of sodium benzoate, compositions with the concentration shown in Table 4 were prepared. The concentration of opiranserin hydrochloride was selected considering the packaging unit of the finished product and the convenience of preparing the IV infusion dilution to be administered to patients. To prepare each composition, 50 mL of water for injection was added to a 200 mL beaker, and the corresponding amount of sodium benzoate was added thereto and stirred at room temperature to completely dissolve it. Next, the corresponding amount of opiranserin hydrochloride was added and stirred at 40°C for 1 hour to completely dissolve it, and then the volume was adjusted to 100 mL using water for injection. The prepared compositions were filtered using a 0.2 µm syringe filter and dispensed into vials for injection.

**[Table 4]**

| | Opiranserin hydrochloride (O) | | Sodium benzoate (B) | | Molar ratio (B/O) |
|---|---|---|---|---|---|
| No. | Concentration (mg/mL) | Concentration (mol/L) | Concentration (mg/mL) | Concentration (mol/L) | |
| Composition 23 | 202 | 0.469 | 60.0 | 0.416 | 0.889 |
| Composition 24 | 202 | 0.469 | 65.0 | 0.451 | 0.963 |
| Composition 25 | 202 | 0.469 | 70.0 | 0.486 | 1.037 |
| Composition 26 | 202 | 0.469 | 75.0 | 0.520 | 1.111 |
| Composition 27 | 202 | 0.469 | 80.0 | 0.555 | 1.185 |
| Composition 28 | 202 | 0.469 | 90.0 | 0.625 | 1.333 |
| Composition 29 | 202 | 0.469 | 100.1 | 0.695 | 1.482 |
| Composition 30 | 169 | 0.392 | 60.1 | 0.417 | 1.063 |
| Composition 31 | 169 | 0.392 | 70.1 | 0.486 | 1.240 |
| Composition 32 | 169 | 0.392 | 80.1 | 0.556 | 1.417 |
| Composition 33 | 169 | 0.392 | 83.7 | 0.581 | 1.482 |
| Composition 34 | 169 | 0.392 | 90.1 | 0.625 | 1.594 |
| Composition 35 | 169 | 0.392 | 100.1 | 0.695 | 1.771 |
| Composition 36 | 102 | 0.336 | 50.5 | 0.169 | 1.482 |
| Composition 37 | 145 | 0.336 | 24.4 | 0.337 | 0.502 |
| Composition 38 | 145 | 0.336 | 48.6 | 0.500 | 1.001 |
| Composition 39 | 145 | 0.336 | 72.0 | 0.891 | 1.482 |
| Composition 40 | 145 | 0.295 | 128.4 | 0.147 | 2.064 |
| Composition 41 | 127 | 0.295 | 21.2 | 0.295 | 0.500 |
| Composition 42 | 127 | 0.295 | 42.5 | 0.436 | 1.001 |
| Composition 43 | 127 | 0.295 | 62.9 | 0.695 | 1.482 |
| Composition 44 | 127 | 0.262 | 100.1 | 0.132 | 2.357 |
| Composition 45 | 113 | 0.262 | 19.0 | 0.262 | 0.502 |
| Composition 46 | 113 | 0.262 | 37.8 | 0.389 | 1.001 |
| Composition 47 | 113 | 0.262 | 56.0 | 0.541 | 1.482 |
| Composition 48 | 113 | 0.237 | 78.0 | 0.702 | 2.649 |
| Composition 49 | 102 | 0.237 | 101.1 | 0.350 | 2.963 |

The compositions in Table 4 filled in vials for injection were stored at room temperature and refrigerated conditions, respectively, and the dissolution stability of opiranserin hydrochloride at each storage temperature was evaluated in each composition. The results are represented in Table 5.

**[Table 5]**

| No. | Property stability (storage at room temperature) | | | | | Property stability (storage in refrigerated condition) | | | | |
|---|---|---|---|---|---|---|---|---|---|---|
| Storage period | 1 week | 2 weeks | 8 weeks | 12 weeks | 16 weeks | 1 week | 2 weeks | 8 weeks | 12 weeks | 16 weeks |
| Composition 23 | ○ | ○ | X | | | X | | | | |
| Composition 24 | ○ | ○ | ○ | ○ | ○ | X | | | | |
| Composition 25 | ○ | ○ | ○ | ○ | ○ | X | | | | |
| Composition 26 | ○ | ○ | ○ | ○ | ○ | X | | | | |
| Composition 27 | ○ | ○ | ○ | ○ | ○ | X | | | | |
| Composition 28 | ○ | ○ | ○ | ○ | ○ | ○ | X | | | |
| Composition 29 | ○ | ○ | ○ | ○ | ○ | ○ | ○ | ○ | ○ | ○ |
| Composition 30 | ○ | ○ | ○ | ○ | ○ | X | | | | |
| Composition 31 | ○ | ○ | ○ | ○ | ○ | ○ | X | | | |
| Composition 32 | ○ | ○ | ○ | ○ | ○ | ○ | ○ | X | | |
| Composition 33 | ○ | ○ | ○ | ○ | ○ | ○ | ○ | ○ | ○ | ○ |
| Composition 34 | ○ | ○ | ○ | ○ | ○ | ○ | ○ | X | | |
| Composition 35 | X | | | | | X | | | | |
| Composition 36 | ○ | ○ | ○ | ○ | ○ | ○ | ○ | ○ | ○ | ○ |
| Composition 37 | X | | | | | X | | | | |
| Composition 38 | ○ | ○ | ○ | ○ | ○ | ○ | ○ | ○ | ○ | ○ |
| Composition 39 | ○ | ○ | ○ | ○ | ○ | ○ | ○ | ○ | ○ | ○ |
| Composition 40 | ○ | ○ | ○ | ○ | ○ | ○ | ○ | ○ | ○ | ○ |
| Composition 41 | X | | | | | X | | | | |
| Composition 42 | ○ | ○ | ○ | ○ | ○ | ○ | ○ | X | | |
| Composition 43 | ○ | ○ | ○ | ○ | ○ | ○ | ○ | ○ | ○ | ○ |
| Composition 44 | ○ | ○ | ○ | ○ | ○ | ○ | ○ | ○ | ○ | ○ |
| Composition 45 | ○ | ○ | ○ | ○ | ○ | ○ | X | | | |
| Composition 46 | ○ | ○ | ○ | ○ | ○ | ○ | ○ | ○ | ○ | ○ |
| Composition 47 | ○ | ○ | ○ | ○ | ○ | ○ | ○ | ○ | ○ | ○ |
| Composition 48 | ○ | ○ | ○ | ○ | ○ | ○ | ○ | ○ | ○ | ○ |
| Composition 49 | ○ | ○ | ○ | ○ | ○ | ○ | ○ | ○ | ○ | ○ |

| | | | | | | | | | | |
|---|---|---|---|---|---|---|---|---|---|---|
| ∘: Clear and transparent liquid, X: Recrystallization generation | | | | | | | | | | |

### Experimental Example 5: Preparation of intravenous infusion (IV infusion) diluent Comparative Example

The opiranserin product currently used clinically is a concentrated composition, in which opiranserin hydrochloride is at a concentration of 10 mg/mL using conventional water for injection and is filled in vials in units of 100 mL, considering the basic solubility of opiranserin hydrochloride and to ensure stability during the product distribution period. In clinical practice, opiranserin is administered as an intravenous infusion to patients with postoperative pain. Before administration, the process of preparing the infusion diluent is required as follows.

First, 100 mL of saline solution is removed from a commercially available 500 mL saline solution bag using a sterilized syringe. Using a new syringe, the concentrated composition is taken from the vial filled with opiranserin and added to the saline solution bag for a total of 100 mL. The contents are thoroughly mixed, and the obtained solution is used as an infusion diluent. In the addition step, if a conventional syringe with a capacity of less than 100 mL is used, the concentrated composition must be taken up with the syringe several times. In this case, close attention should be paid to add the amount accurately and quickly while maintaining sterility.

### Examples 1 and 2

In Examples 1 and 2, concentrated compositions prepared at high concentrations of 102 mg/mL and 202 mg/mL, respectively, were used. As it is manufactured at a high concentration, the final volume of the product can be reduced to 10 mL to 5 mL, so it can be used by filling a vial or a sterilized pre-filled syringe.

The products of Examples 1 and 2 do not require the process of removing part of the saline solution from the saline solution bag in the process of preparing the diluent for intravenous infusion administration to the patient, and the IV infusion diluent can be prepared by injecting only once with a regular syringe. Therefore, it is possible to manufacture quickly and accurately, and sterilization conditions can be guaranteed during the manufacturing process.

**[Table 6] Preparation of finished product**

| | Concentration of opiranserin hydrochloride in finished product | Total volume of finished product administered to patients | Available package formats |
|---|---|---|---|
| Comparative Example | 10 mg/mL | 100 mL | 100 mL vial |
| Example 1 | 102 mg/mL | 10 mL | 10 mL vial or pre-filled syringe |
| Example 2 | 202 mg/mL | 5 mL | 5 mL vial or pre-filled syringe |

**[Table 7] Preparation of infusion diluent**

| | Preparation process for bolus intravenous infusion composition | | | Bolus intravenous infusion composition | |
|---|---|---|---|---|---|
| | Volume of saline solution bag | Removal amount of saline solution | Added amount of finished product | Final volume | Final concentration of opiranserin hydrochloride |
| Comparative Example | 500 mL | 100 mL | 100 mL | 500 mL | 2 mg/mL |
| Example 1 | 500 mL | 0 mL | 10 mL | 510 mL | 2 mg/mL |
| Example 2 | 500 mL | 0 mL | 5 mL | 505 mL | 2 mg/mL |

### Experimental Example 6: Measurement for plasma concentration of high-concentration opiranserin hydrochloride injection

To confirm the equivalence of the infusion diluents of Comparative Example and Examples 1 and 2, plasma concentrations were analyzed using male SD rats. The infusion diluents prepared in Experimental Example 5 were administered to male SD rats at a flow rate of 1 mL/hour for 4 hours, and blood was collected 1, 2, 4, 5, 6, 8 and 28 hours after the start of administration. Then, QTRAP 4500 tandem mass spectrometer (LC-MS/MS)(AB Sciex Pte. Ltd.) was used to analyze the plasma concentration and the results are represented in Figure 1. When blood was collected 1 hour after infusion administration, the plasma concentration was 1,500 to 3,000 ng/mL, and when blood was collected 4 hours after administration, the blood concentration was less than 5,500 ng/mL. Both Examples 1 and 2 showed the same plasma concentration as Comparative Example. As such, it was confirmed that when the high-concentration injection according to the present invention was prepared, it showed equivalent efficacy to the current clinical product.

### Experimental Example 7: Surfactant

When manufacturing high-concentration opiranserin hydrochloride injection using hydrotrope technology, preparation feasibility and stability by a surfactant-which is used as a solubilizing agent in injections-were confirmed.

Each preparation was prepared using the method of Experimental Example 4. After preparation by adding a solubilizing agent that can be conventionally used in injections, the stability was evaluated. The results are represented in Table 9.

**[Table 8] Preparation of finished composition comprising solubilizing agent**

| | Composition 50 | Composition 51 | Composition 52 | Composition 53 | Composition 54 | Composition 55 | Composition 56 |
|---|---|---|---|---|---|---|---|
| Opiranserin hydrochloride | 20.2 g | 20.2 g | 20.2 g | 16.9 g | 16.9 g | 16.9 g | 16.9 g |
| Sodium benzoate | 10.0 g | 10.0 g | 10.0 g | 10.0 g | 10.0 g | 10.0 g | 10.0 g |
| Molar ratio (B/O) | 1.480 | 1.480 | 1.480 | 1.770 | 1.770 | 1.770 | 1.770 |
| Polysorbate 80 | 0.1 g | 0.5 g | 5.0 g | 0.1 g | 0.5 g | 1.0 g | 5.0 g |
| Water for injection | q.s. | q.s. | q.s. | q.s. | q.s. | q.s. | q.s. |
| Total volume | 100 mL | 100 mL | 100 mL | 100 mL | 100 mL | 100 mL | 100 mL |

**[Table 9] Property stability of finished composition comprising solubilizing agent (stored at room temperature/refrigerated condition)**

| No. | Property stability (storage at room temperature) | | | | | Property stability (storage in refrigerated condition) | | | | |
|---|---|---|---|---|---|---|---|---|---|---|
| Storage period | 1 week | 2 weeks | 8 weeks | 12 weeks | 16 weeks | 1 week | 2 weeks | 8 weeks | 12 weeks | 16 weeks |
| Composition 50 | ○ | ○ | ○ | ○ | ○ | ○ | ○ | ○ | ○ | ○ |
| Composition 51 | ○ | ○ | ○ | ○ | ○ | ○ | ○ | ○ | ○ | ○ |
| Composition 52 | ○ | ○ | ○ | ○ | ○ | ○ | ○ | X | | |
| Composition 53 | ○ | ○ | ○ | ○ | ○ | ○ | ○ | ○ | ○ | ○ |
| Composition 54 | ○ | ○ | ○ | ○ | ○ | ○ | ○ | ○ | ○ | ○ |
| Composition 55 | ○ | ○ | ○ | ○ | ○ | ○ | ○ | ○ | ○ | ○ |
| Composition 56 | ○ | ○ | ○ | ○ | ○ | ○ | ○ | ○ | ○ | ○ |

| | | | | | | | | | | |
|---|---|---|---|---|---|---|---|---|---|---|
| ∘: Clear and transparent liquid, X: Recrystallization generation | | | | | | | | | | |

As a result of adding polysorbate 80-which is a representative surfactant-as a solubilizing agent to the high-concentration opiranserin injection, the property stability was maintained for a long time over wider range of molar ratios.

## Claims

1. A pharmaceutical composition comprising opiranserin or a pharmaceutically acceptable salt thereof; a carrier selected from sodium benzoate, trisodium citrate, sodium salicylate and a mixture thereof; and a solvent for injection.

2. The pharmaceutical composition according to Claim 1, wherein the content of opiranserin or a pharmaceutically acceptable salt thereof is 0.1 mol/L to 0.7 mol/L based on the total volume of the composition.

3. The pharmaceutical composition according to Claim 2, wherein the content of opiranserin or a pharmaceutically acceptable salt thereof is 0.2 mol/L to 0.7 mol/L based on the total volume of the composition.

4. The pharmaceutical composition according to Claim 1, wherein the pharmaceutically acceptable salt of opiranserin is hydrochloride, phosphate or sulfate.

5. The pharmaceutical composition according to Claim 4, wherein the pharmaceutically acceptable salt of opiranserin is hydrochloride.

6. The pharmaceutical composition according to Claim 1, wherein the content of sodium benzoate is 0.2 mol/L to 2.0 mol/L based on the total volume of the composition.

7. The pharmaceutical composition according to Claim 6, wherein the content of sodium benzoate is 0.26 mol/L to 0.90 mol/L based on the total volume of the composition.

8. The pharmaceutical composition according to Claim 1, wherein the content of trisodium citrate is 0.08 mol/L to 0.35 mol/L based on the total volume of the composition.

9. The pharmaceutical composition according to Claim 1, wherein the content of sodium salicylate is 0.015 mol/L to 0.10 mol/L, based on the total volume of the composition.

10. The pharmaceutical composition according to Claim 1, wherein the molar ratio of opiranserin or a pharmaceutically acceptable salt thereof to sodium benzoate is 1 : 0.5 to 3.0.

11. The pharmaceutical composition according to Claim 10, wherein the molar ratio of opiranserin or a pharmaceutically acceptable salt thereof to sodium benzoate is 1 : 1.0 to 2.4.

12. The pharmaceutical composition according to Claim 11, wherein the molar ratio of opiranserin or a pharmaceutically acceptable salt thereof to sodium benzoate is 1 : 1.4 to 1.6.

13. The pharmaceutical composition according to Claim 12, wherein the molar ratio of opiranserin or a pharmaceutically acceptable salt thereof to sodium benzoate is 1 : 1.48 to 1.49.

14. The pharmaceutical composition according to Claim 1, wherein the solvent for injection is water for injection.

15. A pharmaceutical composition as an analgesic injection, which comprises 0.1 mol/L to 0.7 mol/L of opiranserin or a pharmaceutically acceptable salt thereof based on the total volume of the injection.

16. A pre-filled syringe containing the pharmaceutical composition as defined in any one of Claims 1 to 14.

17. A method for preparing a bolus intravenous infusion composition comprising:
preparing an intravenous administration container (IV container) containing saline solution; and
injecting the pharmaceutical composition as defined in any one of Claims 1 to 14 into the intravenous administration container (IV container) one time.

18. A method for preparing a bolus intravenous infusion composition comprising:
preparing an intravenous administration container (IV container) containing saline solution; and
injecting the pharmaceutical composition filled in the pre-filled syringe as defined Claim 16 into the intravenous administration container (IV container) one time.
